# EUROPEAN PATENT SPECIFICATION

(11) **EP 2 844 064 B1**
(45) Date of publication and mention of the grant of the patent: **05.09.2018**
(21) Application number: 13730330.1
(22) Date of filing: 02.05.2013
(51) Int. Cl.: A01K 67/027, C12N 9/02, C12N 15/877, C12N 15/85, C12N 9/00

(54) **TRANSGENIC ANIMAL MODEL OF AMYOTROPHIC LATERAL SCLEROSIS**
TRANSGENES TIERMODELL FÜR AMYOTROPHE LATERALSKLEROSE
MODÈLE D'ANIMAL TRANSGÉNIQUE DE SCLÉROSE LATÉRALE AMYOTROPHIQUE

(30) Priority: 04.05.2012 IT TO20120402
(43) Date of publication of application: 11.03.2015
(73) Proprietor: Istituto Zooprofilattico Sperimentale del Piemonte, Liguria e Valle d'Aosta, 10154 Torino (IT); Avantea S.r.l., 26100 Cremona (IT)
(72) Inventor: CASALONE, Cristina, I-10132 Torino (IT); GALLI, Cesare, I-26100 Cremona (IT); CHIEPPA, Maria Novella, I-10148 Torino (IT); CORONA, Cristiano, I-10060 San Secondo di Pinerolo (TO) (IT); BENDOTTI, Caterina, I-20014 Nerviano (MI) (IT); PEROTA, Andrea, I-21017 Samarate (VA) (IT); LAZZARI, Giovanna, I-26100 Cremona (IT); CARAMELLI, Maria, I-10123 Torino (IT); LAGUTINA, Irina, I-26100 Cremona (IT)
(74) Representative: Freyria Fava, Cristina
(86) International application number: PCT/IB2013/053494
(87) International publication number: WO 2013/164792

(56) References cited:
- WO-A2-2007/124751
- M N CHIEPPA ET AL: "Creation of a ubiquitous vector for expression of hSOD1G93A in pigs", TRANSGENIC RESEARCH, vol. 19, 1 April 2010 (2010-04-01), page 326, XP055050423, cited in the application
- WATANABE S ET AL: "A Novel Method for the Production of Transgenic Cloned Pigs: Electroporation-Mediated Gene Transfer to Non-Cultured Cells and Subsequent Selection with Puromycin 1", BIOLOGY OF REPRODUCTION, NEW YORK, NY [U.A.] : ACADEM. PRESS, US, vol. 72, 1 January 2005 (2005-01-01), pages 309-315, XP003024055, ISSN: 0006-3363, DOI: 10.1095/BIOLREPROD.104.031591
- D. YANG ET AL: "Expression of Huntington's disease protein results in apoptotic neurons in the brains of cloned transgenic pigs", HUMAN MOLECULAR GENETICS, vol. 19, no. 20, 15 October 2010 (2010-10-15), pages 3983-3994, XP055006401, ISSN: 0964-6906, DOI: 10.1093/hmg/ddq313
- PETER M. KRAGH ET AL: "Hemizygous minipigs produced by random gene insertion and handmade cloning express the Alzheimer's disease-causing dominant mutation APPsw", TRANSGENIC RESEARCH, vol. 18, no. 4, 1 August 2009 (2009-08-01) , pages 545-558, XP055011723, ISSN: 0962-8819, DOI: 10.1007/s11248-009-9245-4
- CESARE GALLI ET AL: "Genetic engineering including superseding microinjection: new ways to make GM pigs", XENOTRANSPLANTATION, vol. 17, no. 6, 1 November 2010 (2010-11-01), pages 397-410, XP055007654, ISSN: 0908-665X, DOI: 10.1111/j.1399-3089.2010.00590.x
- YAQIONG HUANG ET AL: "Prospect of nuclear transfer technology in mammal animals", REMOTE SENSING, ENVIRONMENT AND TRANSPORTATION ENGINEERING (RSETE), 2011 INTERNATIONAL CONFERENCE ON, IEEE, 24 June 2011 (2011-06-24), pages 7735-7737, XP031906976, DOI: 10.1109/RSETE.2011.5966168 ISBN: 978-1-4244-9172-8
- Randall S Prather ET AL: "Genetically Modified Pigs for Medicine and Agriculture" In: "Biotechnology and Genetic Engineering Reviews", 1 January 2008 (2008-01-01), Nottingham University Press, XP055050381, vol. 25, pages 245-266,
- E. BENDIXEN ET AL: "Advances in porcine genomics and proteomics--a toolbox for developing the pig as a model organism for molecular biomedical research", BRIEFINGS IN FUNCTIONAL GENOMICS, vol. 9, no. 3, 1 May 2010 (2010-05-01), pages 208-219, XP055050637, ISSN: 2041-2649, DOI: 10.1093/bfgp/elq004
- MARIA N CHIEPPA ET AL: "Generation of pre-implantation pig SCNT embryos harboring the amyotrophic lateral sclerosis related hSOD1G93A gene", PRION, vol. 6, no. S, 1 June 2012 (2012-06-01), page 84, XP055050413,
- HUAQIANG YANG ET AL: "Species-dependent neuropathology in transgenic SOD1 pigs", CELL RESEARCH - XIBAO YANJIU, vol. 24, no. 4, 28 February 2014 (2014-02-28), pages 464-481, XP55273523, GB, CN ISSN: 1001-0602, DOI: 10.1038/cr.2014.25

### SEQUENCE LISTING

<110> Istituto Zooprofilattico Sperimentale del Piemonte, Liguria e Valle d'Aosta Avantea S.r.l.
<120> Novel transgenic animal model of Amyotrophic Lateral Sclerosis
<130> BWO15409-CF
<160> 1
<170> PatentIn version 3.5
<210> 1
   <211> 12882
   <212> DNA
   <213> artificial
<220>
   <223> pMG5'3'MARPuro5171-hSOD1G93A vector
<400> 1
   ttgtacaaag tggtgatatc ctcgagtgat ctttttccct ctgccaaaaa ttatggggac      60
   atcatgaagc cccttgagca tctgacttct ggctaataaa ggaaatttat tttcattgca     120
   atagtgtgtt ggaatttttt gtgtctctca ctcggaagga catatgggag ggcaaatcat     180
   ttaaaacatc agaatgagta tttggtttag agtttggcaa catatgccat atgctggctg     240
   ccatgaacaa aggtggctat aaagaggtca tcagtatatg aaacagcccc ctgctgtcca     300
   ttccttattc catagaaaag ccttgacttg aggttagatt ttttttatat tttgttttgt      360
   gttatttttt tctttaacat ccctaaaatt ttccttacat gttttactag ccagattttt     420
   cctcctctcc tgactactcc cagtcatagc tgtccctctt ctcttatgaa gatccctcga     480
   cctgcagccc gggggatcca taatataact gtaccaggtt ttggtttatt acatgtgact     540
   gacggcttcc tatgcgtgct cagaaaacgg cagttgggca ctgcactgcc cggtgatggt     600
   gccacggtgg ctcctgccgc cttctttgat attcactctg ttgtatttca tctcttgttg     660
   ccgatgaaag gatataacag tctctgagga aatacttggt atttcttctg atcagcgttt     720
   ttataagtaa tgttgaatat tggataaggc tgtgtgtcct ttgtcttggg agacaaagcc     780
   cacagcaggt ggtggttggg tggtggcagc tcagtgacag gagaggtttt tttgcctgtt     840
   ttttttgttg tttttttttt ttaagtaagg tgttcttttt tcttagtaaa atttctactg      900
   gactgtatgt tttgacaggt cagaaacatt tcttcaaaag aagaaccttt tggaaactgt     960
   acagcccttt tctttcattc cctttttgct ttctgtgcca atgcctttgg ttctgattgc     1020
   attatggaaa acgttgatcg gaacttgagg tttttattta tagtgtggct tgaaagcttg    1080
   gatagctgtt gttacatgag ataccttatt aagtttaggc cagcttgatg ctttattttt    1140
   tttcctttga agtagtgagc gttctctggt ttttttcctt tgaaactggc gaggcttaga    1200
   tttttctaat gggatttttt acctgatgat ctagttgcat acccaaatgc ttgtaaatgt    1260
   tttcctagtt aacatgttga taacttcgga tttacatgtt gtatatactt gtcatctgtg    1320
   tttctagtaa aaatatatgg catttataga aatacgtaat tcctgatttc cttttttttt    1380
   tatctctatg ctctgtgtgt acaggtcaaa cagacttcac tcctattttt atttatagaa    1440
   ttttatatgc agtctgtcgt tggttcttgt gttgtaagga tacagcctta aatttcctag    1500
   agcgatgctc agtaaggcgg gttgtcacat gggttcaaat gtaaaacggg cacgtttgct    1560
   gctgccttcc cagatccagg acactaaact gcttctgcaa ctgaggtata aatcgcttca    1620
   gatcccagga agtgtagatc cacgtgcata ttcttaaaga agaatgaata ctttctaaaa    1680
   tatgttggca taggaagcaa gctgcatgga tttatttggg acttaaatta ttttggtaac    1740
   ggagtgcata ggttttaaac acagttgcag catgctaacg agtcacagca tttatgcaga    1800
   agtgatgcct gttgcagctg tttacggcac tgccttgcag tgagcattgc agataggggt    1860
   ggggtgcttt gtgtcgtgtt gggacacgct gccacacagc cacctcccga acatatctca    1920
   cctgctgggt acttttcaaa ccatcttagc agtagtagat gagttactat gaaacagaga    1980
   agttcctcag ttggatattc tcatgggatg tcttttttcc catgttgggc aaagtatgat      2040
   aaagcatctc tatttgtaaa ttatgcactt gttagttcct gaatcctttc tatagcacca    2100
   cttattgcag caggtgtagg ctctggtgtg gcctgtgtct gtgcttcaat cttttaagct    2160
   tctttggaaa tacaccgact tgattgaagt ctcttgaaga tagtaaacag tacttacctt    2220
   tgatcccaat gaaatcgagc atttcagttg taaaagaatt ccgcctattc ataccatgta    2280
   atgtaatttt acacccccag tgctgacact ttggaatata ttcaagtaat agactttggc   2340
   ctcaccctct tgtgtactgt attttgtaat agaaaatatt ttaaactgtg catatgatta    2400
   ttacattatg aaagagacat tctgctgatc ttcaaatgta agaaaatgag gagtgcgtgt    2460
   gcttttataa atacaagtga ttgcaaatta gtgcaggtgt ccttaaaaaa aaaaaaaagt    2520
   aatataaaaa ggaccaggtg ttttacaagt gaaatacatt cctatttgga aaacagttac    2580
   atttttatga agattaccag cgcttgctga ctttctaaac ataaggctgt attgtcttcc     2640
   tgtaccattg catttcctca ttcccaattt gcacaaggat gtctgggtaa actattcaag     2700
   aaatggcttt gaaatacagc atgggagctt gtctgagttg gaatgcagag ttgcactgca     2760
   aaatgtcagg aaatggatgt ctctcagaat gcccaactcc aaaggattta tatgtgtata     2820
   tagtaagcag tttcctgatt ccagcaggcc aaagagtctg ctgaatgttg cgttgccgga     2880
   gacctgtatt tctcaacaag gtaagatggt atcctagcaa ctgcggattt taatacattt     2940
   tcagcagaag tacttagtta atctctacct ttagggatcg tttcatcatt tttagatgtt     3000
   atacttgaaa tactgcataa cttttagctt tcatgggttc ctttttttca gcctttagga     3060
   gactgttaag caatttgctg tccaactttt gtgttggtct taaactgcaa tagtagttta     3120
   ccttgtattg aagaaataaa gaccattttt atattaaaaa atacttttgt ctgtcttcat     3180
   tttgacttgt ctgatatcct tgcagtgctc attatgtcag ttctgtcaga tattcacaca     3240
   tcaaaactta acgtgagctc agtggagtta cagctgcggt tttgatgctg ttattatttc     3300
   tgaaactaga aatgatgttg tcttcatctg ctcatcaaac acttcatgca gcagtttaag     3360
   gctagtgaga aatgcataca tttattgata cttttttaaa gtcaactttt tatcagattt     3420
   ttttttcatt tggaaatata ttgttttcta gagcggccgc caccgcggtg gagctccagc     3480
   ttttgttccc tttagtgagg gttaattgcg cgcttggcgt aatcatggtc atagctgttt     3540
   cctgtgtgaa attgttatcc gctcacaatt ccacacaaca tacgagccgg aagcataaag     3600
   tgtaaagcct ggggtgccta atgagtgagc taactcacat taattgcgtt gcgctcactg     3660
   cccgctttcc agtcgggaaa cctgtcgtgc cagctgcatt aatgaatcgg ccaacgcgcg     3720
   gggagaggcg gtttgcgtat tgggcgctct tccgcttcct cgctcactga ctcgctgcgc     3780
   tcggtcgttc ggctgcggcg agcggtatca gctcactcaa aggcggtaat acggttatcc     3840
   acagaatcag gggataacgc aggaaagaac atgtgagcaa aaggccagca aaaggccagg     3900
   aaccgtaaaa aggccgcgtt gctggcgttt ttccataggc tccgcccccc tgacgagcat     3960
   cacaaaaatc gacgctcaag tcagaggtgg cgaaacccga caggactata aagataccag     4020
   gcgtttcccc ctggaagctc cctcgtgcgc tctcctgttc cgaccctgcc gcttaccgga     4080
   tacctgtccg cctttctccc ttcgggaagc gtggcgcttt ctcatagctc acgctgtagg     4140
   tatctcagtt cggtgtaggt cgttcgctcc aagctgggct gtgtgcacga accccccgtt     4200
   cagcccgacc gctgcgcctt atccggtaac tatcgtcttg agtccaaccc ggtaagacac     4260
   gacttatcgc cactggcagc agccactggt aacaggatta gcagagcgag gtatgtaggc     4320
   ggtgctacag agttcttgaa gtggtggcct aactacggct acactagaag gacagtattt     4380
   ggtatctgcg ctctgctgaa gccagttacc ttcggaaaaa gagttggtag ctcttgatcc     4440
   ggcaaacaaa ccaccgctgg tagcggtggt ttttttgttt gcaagcagca gattacgcgc     4500
   agaaaaaaag gatctcaaga agatcctttg atcttttcta cggggtctga cgctcagtgg     4560
   aacgaaaact cacgttaagg gattttggtc atgagattat caaaaaggat cttcacctag     4620
   atccttttaa attaaaaatg aagttttaaa tcaatctaaa gtatatatga gtaaacttgg     4680
   tctgacagtt accaatgctt aatcagtgag gcacctatct cagcgatctg tctatttcgt     4740
   tcatccatag ttgcctgact ccccgtcgtg tagataacta cgatacggga gggcttacca     4800
   tctggcccca gtgctgcaat gataccgcga gacccacgct caccggctcc agatttatca     4860
   gcaataaacc agccagccgg aagggccgag cgcagaagtg gtcctgcaac tttatccgcc     4920
   tccatccagt ctattaattg ttgccgggaa gctagagtaa gtagttcgcc agttaatagt     4980
   ttgcgcaacg ttgttgccat tgctacaggc atcgtggtgt cacgctcgtc gtttggtatg     5040
   gcttcattca gctccggttc ccaacgatca aggcgagtta catgatcccc catgttgtgc     5100
   aaaaaagcgg ttagctcctt cggtcctccg atcgttgtca gaagtaagtt ggccgcagtg     5160
   ttatcactca tggttatggc agcactgcat aattctctta ctgtcatgcc atccgtaaga     5220
   tgcttttctg tgactggtga gtactcaacc aagtcattct gagaatagtg tatgcggcga     5280
   ccgagttgct cttgcccggc gtcaatacgg gataataccg cgccacatag cagaacttta     5340
   aaagtgctca tcattggaaa acgttcttcg gggcgaaaac tctcaaggat cttaccgctg     5400
   ttgagatcca gttcgatgta acccactcgt gcacccaact gatcttcagc atcttttact     5460
   ttcaccagcg tttctgggtg agcaaaaaca ggaaggcaaa atgccgcaaa aaagggaata     5520
   agggcgacac ggaaatgttg aatactcata ctcttccttt ttcaatatta acgcttacaa     5580
   tttccattcg ccattcaggc tgcgcaactg ttgggaaggg cgatcggtgc gggcctcttc     5640
   gctattacgc cagctggcga aagggggatg tgctgcaagg cgattaagtt gggtaacgcc     5700
   agggttttcc cagtcacgac gttgtaaaac gacggccagt gagcgcgcgt aatacgactc     5760
   actatagggc gaattgggta ccataacttc gtatagtaca cattatacga agttatgtcg     5820
   atcgacggta tcgataagct tgatatcgaa ttgttgttgt taacttgttt attgcagctt     5880
   ataatggtta caaataaagc aatagcatca caaatttcac aaataaagca tttttttcac     5940
   tgcattctag ttgtggtttg tccaaactca tcaatgtatc ttatcatgtc tggatctcgc     6000
   gcgccaggct gtggaatgtg tgtcagttag ggtgtggaaa gtccccaggc tccccagcag     6060
   gcagaagtat gcaaagcatg catctcaatt agtcagcaac caggtgtgga aagtccccag     6120
   gctccccagc aggcagaagt atgcaaagca tgcatctcaa ttagtcagca accatagtcc     6180
   cgcccctaac tccgcccatc ccgcccctaa ctccgcccag ttccgcccat tctccgcccc     6240
   atggctgact aatttttttt atttatgcag aggccgaggc cgcctcggcc tctgagctat     6300
   tccagaagta gtgaggaggc ttttttggag gccgtcggcc gccacgaccg gtgccgccac     6360
   catcccctga cccacgcccc tgacccctca caaggagacg accttccatg accgagtaca     6420
   agcccacggt gcgcctcgcc acccgcgacg acgtcccccg ggccgtacgc accctcgccg     6480
   ccgcgttcgc cgactaccct gcaacacgcc atacagtgga ccctgaccgc cacatcgagc     6540
   gggtcaccga gctgcaagaa ctcttcctca cgcgcgtcgg gctcgacatc ggcaaggtgt     6600
   gggtcgcgga cgacggcgcc gcggtggcgg tctggaccac gccggagagc gtcgaagcgg     6660
   gggcggtgtt cgccgagatc ggcccgcgca tggccgagtt gagcggttcc cggctggccg     6720
   cgcagcaaca gatggaaggc ctcctggcgc cgcaccggcc caaggagccc gcgtggttcc     6780
   tggccaccgt cggcgtctcg cccgaccacc agggcaaggg tctgggcagc gccgtcgtgc     6840
   tccccggagt ggaggcggcc gagcgcgccg gggtgcccgc cttcctggag acctccgcgc     6900
   cccgcaacct ccccttctac gagcggctcg gcttcaccgt caccgccgac gtcgagtgcc     6960
   cgaaggaccg cgcgacctgg tgcatgaccc gcaagcccgg tgcctgacgc ccgccccacg     7020
   acccgcagcg cccgaccgaa aggagcgcac gaccccatgg ctccgaccga agccacccgg     7080
   ggcggccccg ccgaccccgc acccgccccc gaggcccacc gactctagag ctcgctgatc     7140
   agcctcgact gtgcctttct agttgccagc catctgttgt ttgcccctcc cccgtgcctt     7200
   ccttgaccct ggaaggtgcc actcccactg tcctttccta ataaaatgag gaaattgcat     7260
   cgcattgtct gagtaggtgt cattctattc tggggggtgg ggtggggcag gacagcaagg     7320
   gggaggattg ggaagacaat agcaggcatg ctggggatgc ggtgggctct atggcttctg     7380
   aggcggaaag aaccagctgg ggctcgagat ccactagttc tagtataact tcgtatagta     7440
   cacattatac gaagttatgc ggccgctcta gaagtactct cgagaagctt tttgaattct     7500
   ttggatccat aatataactg taccaggttt tggtttatta catgtgactg acggcttcct     7560
   atgcgtgctc agaaaacggc agttgggcac tgcactgccc ggtgatggtg ccacggtggc     7620
   tcctgccgcc ttctttgata ttcactctgt tgtatttcat ctcttgttgc cgatgaaagg     7680
   atataacagt ctctgaggaa atacttggta tttcttctga tcagcgtttt tataagtaat     7740
   gttgaatatt ggataaggct gtgtgtcctt tgtcttggga gacaaagccc acagcaggtg     7800
   gtggttgggt ggtggcagct cagtgacagg agaggttttt ttgcctgttt tttttgttgt     7860
   tttttttttt taagtaaggt gttctttttt cttagtaaaa tttctactgg actgtatgtt     7920
   ttgacaggtc agaaacattt cttcaaaaga agaacctttt ggaaactgta cagccctttt     7980
   ctttcattcc ctttttgctt tctgtgccaa tgcctttggt tctgattgca ttatggaaaa     8040
   cgttgatcgg aacttgaggt ttttatttat agtgtggctt gaaagcttgg atagctgttg     8100
   ttacatgaga taccttatta agtttaggcc agcttgatgc tttatttttt ttcctttgaa     8160
   gtagtgagcg ttctctggtt tttttccttt gaaactggcg aggcttagat ttttctaatg     8220
   ggatttttta cctgatgatc tagttgcata cccaaatgct tgtaaatgtt ttcctagtta     8280
   acatgttgat aacttcggat ttacatgttg tatatacttg tcatctgtgt ttctagtaaa     8340
   aatatatggc atttatagaa atacgtaatt cctgatttcc tttttttttt atctctatgc     8400
   tctgtgtgta caggtcaaac agacttcact cctattttta tttatagaat tttatatgca     8460
   gtctgtcgtt ggttcttgtg ttgtaaggat acagccttaa atttcctaga gcgatgctca     8520
   gtaaggcggg ttgtcacatg ggttcaaatg taaaacgggc acgtttgctg ctgccttccc     8580
   agatccagga cactaaactg cttctgcaac tgaggtataa atcgcttcag atcccaggaa     8640
   gtgtagatcc acgtgcatat tcttaaagaa gaatgaatac tttctaaaat atgttggcat     8700
   aggaagcaag ctgcatggat ttatttggga cttaaattat tttggtaacg gagtgcatag     8760
   gttttaaaca cagttgcagc atgctaacga gtcacagcat ttatgcagaa gtgatgcctg     8820
   ttgcagctgt ttacggcact gccttgcagt gagcattgca gataggggtg gggtgctttg     8880
   tgtcgtgttg ggacacgctg ccacacagcc acctcccgaa catatctcac ctgctgggta     8940
   cttttcaaac catcttagca gtagtagatg agttactatg aaacagagaa gttcctcagt     9000
   tggatattct catgggatgt cttttttccc atgttgggca aagtatgata aagcatctct     9060
   atttgtaaat tatgcacttg ttagttcctg aatcctttct atagcaccac ttattgcagc     9120
   aggtgtaggc tctggtgtgg cctgtgtctg tgcttcaatc ttttaagctt ctttggaaat     9180
   acaccgactt gattgaagtc tcttgaagat agtaaacagt acttaccttt gatcccaatg     9240
   aaatcgagca tttcagttgt aaaagaattc cgcctattca taccatgtaa tgtaatttta     9300
   cacccccagt gctgacactt tggaatatat tcaagtaata gactttggcc tcaccctctt     9360
   gtgtactgta ttttgtaata gaaaatattt taaactgtgc atatgattat tacattatga     9420
   aagagacatt ctgctgatct tcaaatgtaa gaaaatgagg agtgcgtgtg cttttataaa     9480
   tacaagtgat tgcaaattag tgcaggtgtc cttaaaaaaa aaaaaaagta atataaaaag     9540
   gaccaggtgt tttacaagtg aaatacattc ctatttggaa aacagttaca tttttatgaa     9600
   gattaccagc gcttgctgac tttctaaaca taaggctgta ttgtcttcct gtaccattgc     9660
   atttcctcat tcccaatttg cacaaggatg tctgggtaaa ctattcaaga aatggctttg     9720
   aaatacagca tgggagcttg tctgagttgg aatgcagagt tgcactgcaa aatgtcagga     9780
   aatggatgtc tctcagaatg cccaactcca aaggatttat atgtgtatat agtaagcagt     9840
   ttcctgattc cagcaggcca aagagtctgc tgaatgttgc gttgccggag acctgtattt     9900
   ctcaacaagg taagatggta tcctagcaac tgcggatttt aatacatttt cagcagaagt     9960
   acttagttaa tctctacctt tagggatcgt ttcatcattt ttagatgtta tacttgaaat    10020
   actgcataac ttttagcttt catgggttcc tttttttcag cctttaggag actgttaagc    10080
   aatttgctgt ccaacttttg tgttggtctt aaactgcaat agtagtttac cttgtattga    10140
   agaaataaag accattttta tattaaaaaa tacttttgtc tgtcttcatt ttgacttgtc    10200
   tgatatcctt gcagtgctca ttatgtcagt tctgtcagat attcacacat caaaacttaa    10260
   cgtgagctca gtggagttac agctgcggtt ttgatgctgt tattatttct gaaactagaa    10320
   atgatgttgt cttcatctgc tcatcaaaca cttcatgcag cagtttaagg ctagtgagaa    10380
   atgcatacat ttattgatac ttttttaaag tcaacttttt atcagatttt tttttcattt    10440
   ggaaatatat tgttttctag agtcgacatt gattattgac tagttattaa tagtaatcaa    10500
   ttacggggtc attagttcat agcccatata tggagttccg cgttacataa cttacggtaa     10560
   atggcccgcc tggctgaccg cccaacgacc cccgcccatt gacgtcaata atgacgtatg     10620
   ttcccatagt aacgccaata gggactttcc attgacgtca atgggtggac tatttacggt     10680
   aaactgccca cttggcagta catcaagtgt atcatatgcc aagtacgccc cctattgacg     10740
   tcaatgacgg taaatggccc gcctggcatt atgcccagta catgacctta tgggactttc     10800
   ctacttggca gtacatctac gtattagtca tcgctattac catgggtcga ggtgagcccc     10860
   acgttctgct tcactctccc catctccccc ccctccccac ccccaatttt gtatttattt     10920
   attttttaat tattttgtgc agcgatgggg gcgggggggg ggggggcgcg cgccaggcgg     10980
   ggcggggcgg ggcgaggggc ggggcggggc gaggcggaga ggtgcggcgg cagccaatca     11040
   gagcggcgcg ctccgaaagt ttccttttat ggcgaggcgg cggcggcggc ggccctataa     11100
   aaagcgaagc gcgcggcggg cgggagtcgc tgcgttgcct tcgccccgtg ccccgctccg     11160
   cgccgcctcg cgccgcccgc cccggctctg actgaccgcg ttactcccac aggtgagcgg     11220
   gcgggacggc ccttctcctc cgggctgtaa ttagcgcttg gtttaatgac ggctcgtttc     11280
   ttttctgtgg ctgcgtgaaa gccttaaagg gctccgggag ggccctttgt gcggggggga     11340
   gcggctcggg gggtgcgtgc gtgtgtgtgt gcgtggggag cgccgcgtgc ggcccgcgct     11400
   gcccggcggc tgtgagcgct gcgggcgcgg cgcggggctt tgtgcgctcc gcgtgtgcgc     11460
   gaggggagcg cggccggggg cggtgccccg cggtgcgggg gggctgcgag gggaacaaag     11520
   gctgcgtgcg gggtgtgtgc gtgggggggt gagcaggggg tgtgggcgcg gcggtcgggc     11580
   tgtaaccccc ccctgcaccc ccctccccga gttgctgagc acggcccggc ttcgggtgcg     11640
   gggctccgtg cggggcgtgg cgcggggctc gccgtgccgg gcggggggtg gcggcaggtg     11700
   ggggtgccgg gcggggcggg gccgcctcgg gccggggagg gctcggggga ggggcgcggc     11760
   ggccccggag cgccggcggc tgtcgaggcg cggcgagccg cagccattgc cttttatggt     11820
   aatcgtgcga gagggcgcag ggacttcctt tgtcccaaat ctggcggagc cgaaatctgg     11880
   gaggcgccgc cgcaccccct ctagcgggcg cgggcgaagc ggtgcggcgc cggcaggaag     11940
   gaaatgggcg gggagggcct tcgtgcgtcg ccgcgccgcc gtccccttct ccatctccag     12000
   cctcggggct gccgcagggg gacggctgcc ttcggggggg acggggcagg gcggggttcg     12060
   gcttctggcg tgtgaccggc ggctctagag cctctgctaa ccatgttcat gccttcttct     12120
   ttttcctaca gctcctgggc aacgtgctgg ttgttgtgct gtctcatcat tttggcaaag     12180
   aattgcaatt ggatatcaca agtttgtaca aaaaagcagg ctagctcact cattaggcac     12240
   ccgtcgagat cctgcagtcc tcggaaccag gacctcggcg tggcctagcg agttatggcg     12300
   acgaaggccg tgtgcgtgct gaagggcgac ggcccagtgc agggcatcat caatttcgag     12360
   cagaaggaaa gtaatggacc agtgaaggtg tggggaagca ttaaaggact gactgaaggc     12420
   ctgcatggat tccatgttca tgagtttgga gataatacag caggctgtac cagtgcaggt     12480
   cctcacttta atcctctatc cagaaaacac ggtgggccaa aggatgaaga gaggcatgtt     12540
   ggagacttgg gcaatgtgac tgctgacaaa gatgcagtgg ccgatgtgtc tattgaagat     12600
   tctgtgatct cactctcagg agaccattgc atcattggcc gcacactggt ggtccatgaa     12660
   aaagcagatg acttgggcaa aggtggaaat gaagaaagta caaagacagg aaacgctgga     12720
   agtcgtttgg cttgtggtgt aattgggatc gcccaataaa cattcccttg gatgtagtct     12780
   gaggcccctt aactcatctg ttatcctgcg gccgctcgag agctcaggta ccgcggccgc     12840
   gtcgagaatt cccggggctt ttttatgcag gacccagctt tc                        12882

## Description

### FIELD OF THE INVENTION

The present invention concerns a novel transgenic animal as model of Amyotrophic Lateral Sclerosis (ALS).

### BACKGROUND ART

Amyotrophic lateral sclerosis (ALS) is characterized by selective and progressive degeneration of upper and lower motor neurons, leading to muscle weakness, atrophy and evolving to complete paralysis. The disease impact on patients life quality is devastating and death occurs in 2 to 5 years. ALS occurs in two forms: familial (FALS) and sporadic (SALS).

FALS accounts for 5-10% of ALS cases and is related to several gene mutations, mostly inheritable in a dominant manner. Nearly 20% of FALS forms are linked to more than 100 mutations in the Cu/Zn superoxide dismutase (SOD1) gene, all showing dominant hereditary patterns.

A characteristic hallmark of FALS linked to mutant SOD1 is the abnormal accumulation of ubiquitine-immunoreactive misfolded protein in the cytoplasm of degenerating motor neurons. It has been hypothesized that protein misfolding and aggregation may contribute to disease pathogenesis, although a causative role remains controversial and pathogenic mechanisms underlying ALS still remain essentially undisclosed.

Research on ALS mainly relies on experimental animal models like transgenic rats and mice overexpressing mutant human SOD1. Besides rodent models, a variety of animal species such as zebrafish, *Caenorhabditis elegans* and *Drosophila melanogaster* has already been employed to unravel ALS pathogenetic mechanisms, although phylogenetic distance from human species prevents their employment in clinical research.

On the contrary, rats certainly represent an interesting alternative for modelling neurodegenerative disorders as their relative larger size allows some experimental procedures which are not easily carried out in other laboratory animal species. The lack of efficient tools for genetic manipulation however has dramatically limited rat employment as model for human diseases (Bugos et al., 2009).

A great number of murine models carrying a variety of SOD1 mutations has been hitherto produced. Currently, the most widely employed animal model is a transgenic mouse with a glycine to alanin conversion at the 93^{rd} codon (G93A) of the SOD1 gene. These mice reliably reproduce the ALS patients phenotype progression developing a rapidly progressive motor neuron degeneration characterized by limb muscles weakness that evolves to paralysis and premature death four months after symptoms onset (Turner and Talbot, 2008), a too short interval compared to le lifespan of the mouse relative to the onset of the disease in humans. These animal models have certainly facilitated investigations on the selective vulnerability of motor neurons, however doubts have been recently raised about rodents suitability to faithfully reproduce the human disease (Schnabel, 2008; Scott et al., 2008). As a matter of fact, encouraging results of drug tests in rodents have never been so far successfully translated to humans (Benatar, 2007; Van de Bosch, 2011) and in some cases, molecules delaying disease progression in transgenic mice, such as minocycline, have resulted even detrimental in ALS patients (Gordon et al., 2007). Since human and rodent species differ in size, lifespan, physiology, anatomy and biochemical aspects, data extrapolation is difficult.

In the patent literature the international patent application WO-A-2007/124751 concerning a transgenic pig showing ALS phenotype has been identified. Such a transgenic pig has been realized using the Sperm Mediated Gene Transfer (SMGT) technique. Such a technique is nevertheless not efficient. In fact, this technique allows the transgene integration in the host cell but not guarantees its expression as the transgene is rearranged thus preventing the protein expression. Animals obtained with SMGT are often mosaics, namely the integration takes place in different times after fertilization and therefore not all cells are transgenic. Moreover data about expression of the endogenous mutated gene (porcine SOD1 mutated protein) in pig's tissues are missing.

In view of the foregoing drawbacks, a more homologous animal model should be created in an animal evolutionarily closer to human species in order to provide a better tool to study the disease.

### OBJECT AND SUMMARY OF THE INVENTION

Object of the represent invention is to provide a new animal model of ALS which may be used in clinical studies for identification of therapies for human species.

According to the invention, the above object is achieved thanks to the process specified in the ensuing claims, which are understood as forming an integral part of the present description.

In an embodiment, the present disclosure provides for a transgenic swine suitable to develop Amyotrophic Lateral Sclerosis, wherein the swine is transgenic for a vector having the nucleotide sequence set forth in SEQ ID No.: 1 encoding a human Cu/Zn superoxide dismutase gene (SOD1) carrying a glycine to alanine mutation at codon 93.

In a further embodiment the transgenic swine has been obtained by means of i) *in vitro* transfection of somatic cells, preferably fibroblasts, with the exogenous gene and ii) somatic cell nuclear transfer technique, which allows the expression of the exogenous gene in all animal's cells, preferably animal's neuronal cells, thus representing more closely the human pathology.

The instant description discloses the generation of live transgenic Yucatan minipigs expressing the mutated protein codified from the hSOD1-G93A gene and then suitable to develop ALS symptoms.

In a further embodiment, the present disclosure concerns a process for generation of the transgenic swine suitable to develop Amyotrophic Lateral Sclerosis, wherein the swine is transgenic for expressing an exogenous gene preferably in neuronal cells, the exogenous gene being a human SOD1 gene carrying a mutation (wherein the human SOD1 gene is preferably mutated at 93^{rd} codon, with a glycine to alanine conversion). The process comprises the following steps:
a. providing a vector encoding the exogenous gene;
b. providing adult swine fibroblasts;
c. nucleofecting the vector into the fibroblasts, obtaining transgenic fibroblasts;
d. providing at least one enucleated swine oocyte by means of metaphase chromosomes removal from an intact oocyte;
e. contacting the at least one enucleated oocyte with at least one transgenic fibroblast;
f. fusing the at least one enucleated oocyte with at least one transgenic fibroblast obtaining an embryo;
g. cultivating the embryo;
h. implanting at least one cultivated embryo, preferably 70, more preferably 120, to a sow uterus;
i. natural birth or cesarean section delivering of at least one newborn transgenic swine at the end of sow pregnancy,
wherein after step c. analysis of vector expression in transgenic fibroblasts is carried out for selecting transgenic fibroblasts having uniform and intensive expression of the vector, wherein step e. is carried out using the selected transgenic fibroblasts, wherein the vector encoding the exogenous gene is carrying the selectable marker Puromycin, and wherein the vector encoding the exogenous gene has the nucleotide sequence set forth in SEQ ID No.:1.

In further embodiments, the transgenic swine herein described can be used for screening compounds pharmaceutically active in the treatment of human ALS and/or as a model for the study of human ALS.

### BRIEF DESCRIPTION OF THE DRAWINGS

The invention will now be described, by way of example only, with reference to the enclosed figures of drawing, wherein:

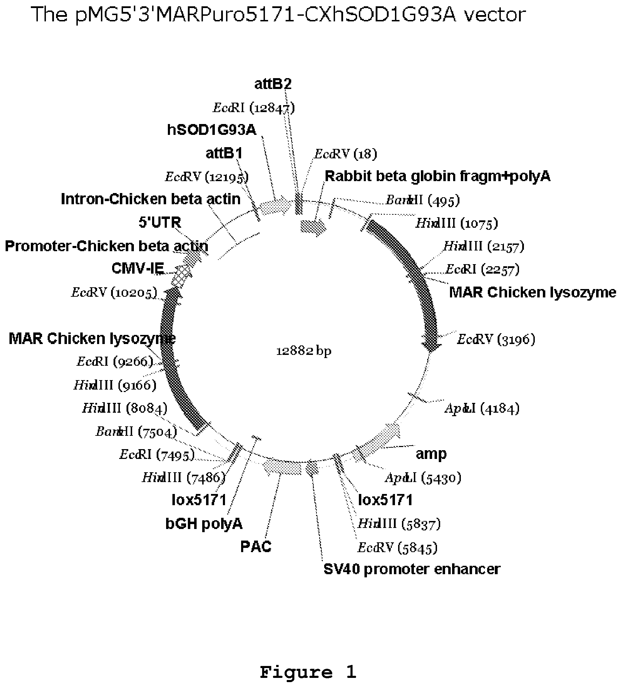

- **FIG. 1****: the pMG5'3'MARPuro5171-hSOD1G93A structure in a graphical representation.**

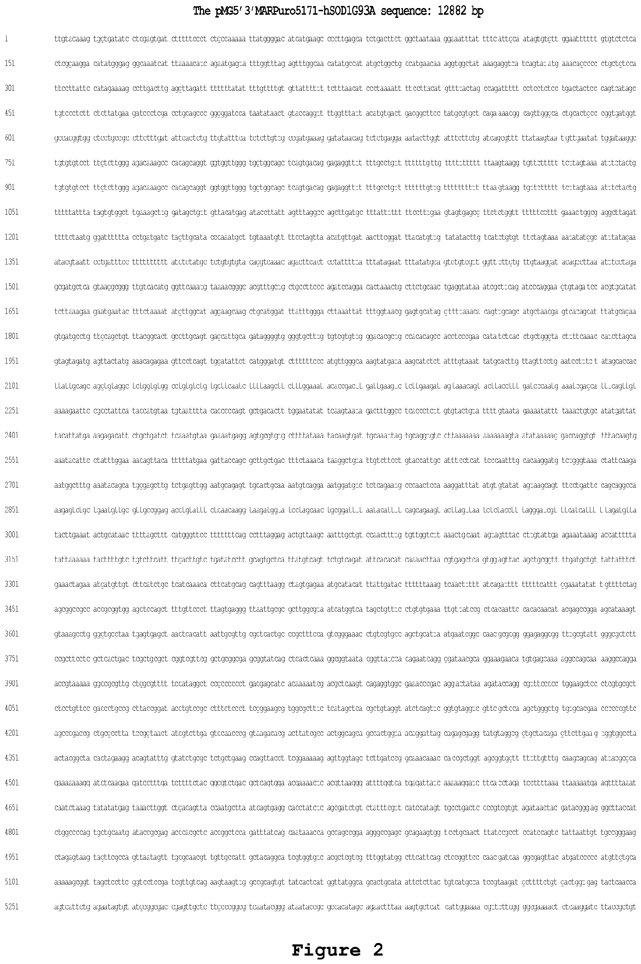

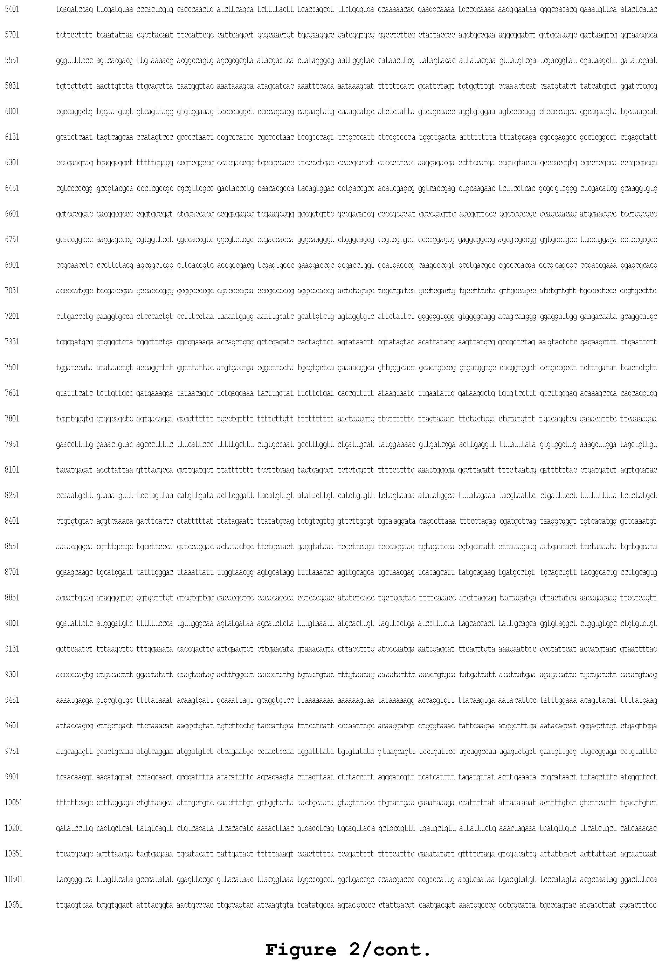

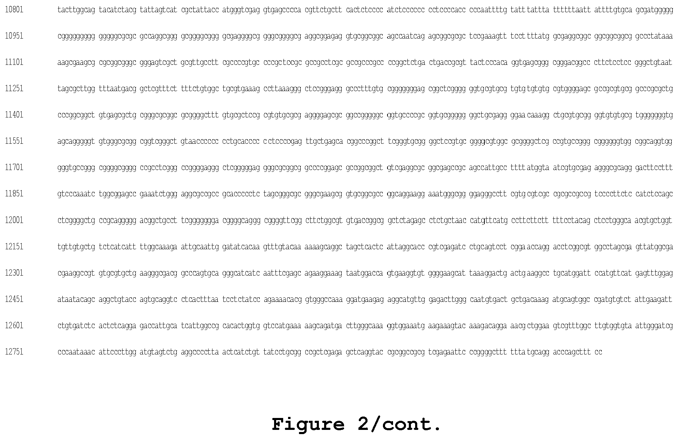

- **FIG. 2****: Complete sequence of the pMG5'3'MARPuro5171-hSOD1G93A vector.**

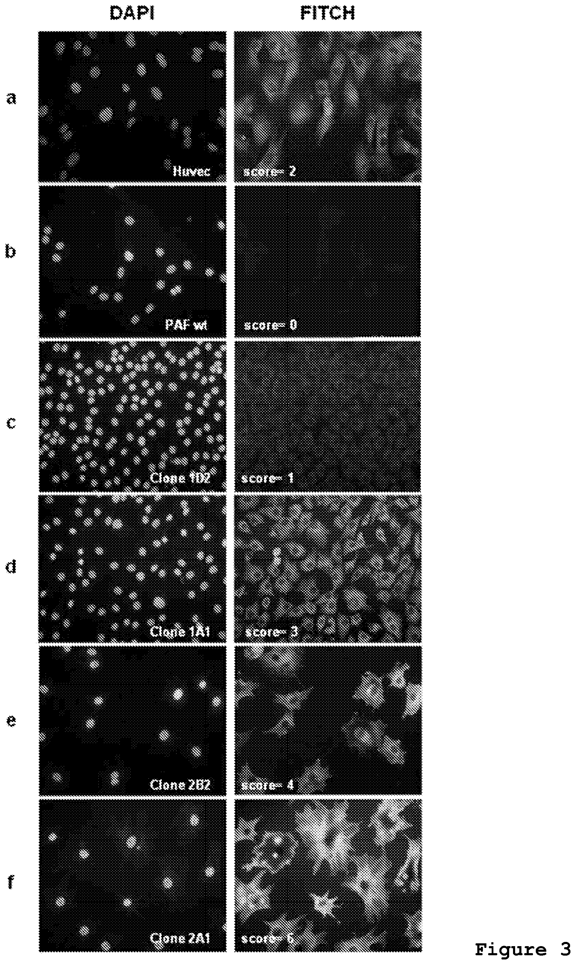

- **FIG. 3****: ICC on PAF (pig adult fibroblasts) clones transfected with the pMG5'3'MARPuro5171-hSOD1-G93A vector.** [a] Human Umbilical Vein Endothelial Cells (Huvec) used as positive control, with score=2 of transgene expression level; [b] wild-type PAF used as negative control (score=0); [c] PAF clone 1D2 with score=1; [d] PAF clone 1A1 with score=3; [e] PAF clone 2B2 with scores=4 and [f] clone 2A1 with a score=6. DAPI is the filter used to highlight the Hoechst nuclear staining to identify the nucleus of the cells. FITCH allows the detection of the fluorophore conjugated with the secondary anti-rabbit antibody and then the transgene expression.

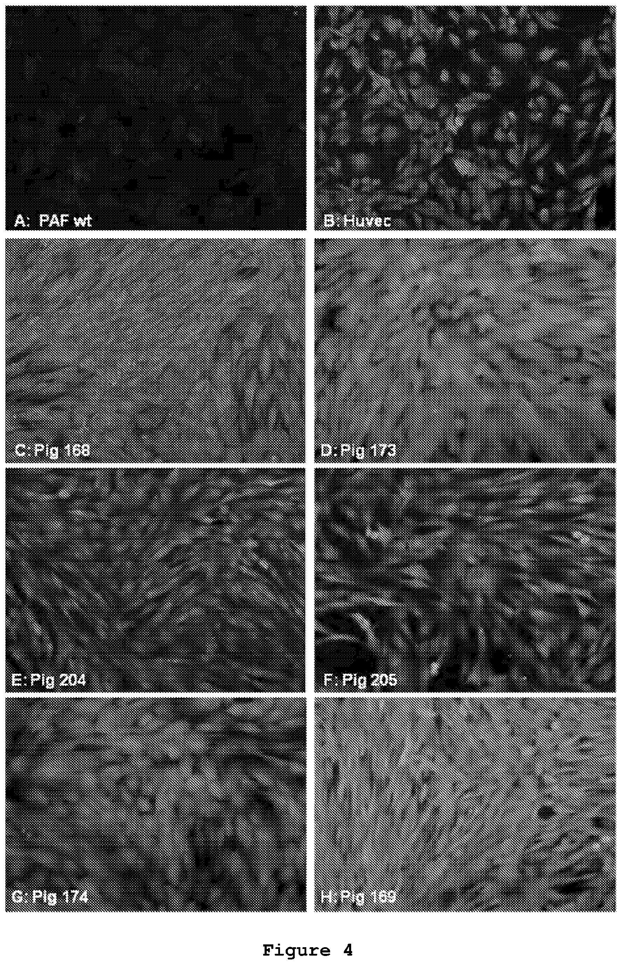

- **FIG. 4****: ICC on PAF biopsied from the 5 transgenic living pigs. ICC on PAF obtained from ear biopsy of the 5 transgenic living pigs.** Picture shows FITCH signal obtained with Millipore 07-403 antibody staining. hSOD1-G93A expression is revealed in all animals analyzed. [A] and [B] display staining in wild-type PAF and Huvec cells, respectively used as negative and positive controls. [C] Pig 168 is characterized by a moderate cytoplasmic and perinuclear staining. [D] Pig 173 displays an intense cytoplasmic labelling along with a perinuclear ring, noticeable in some cells. [E] and [F] show nuclear labelling with faint cytoplasmic staining detected in pigs 204 and 205 respectively. [G] Pig 174 displays a faint cytoplasmic staining. Perinuclear rings and faint nuclei labelling can also be observed in some cells. [H] shows intense cytoplasmic labelling detected in cells from stillborn piglets 169.

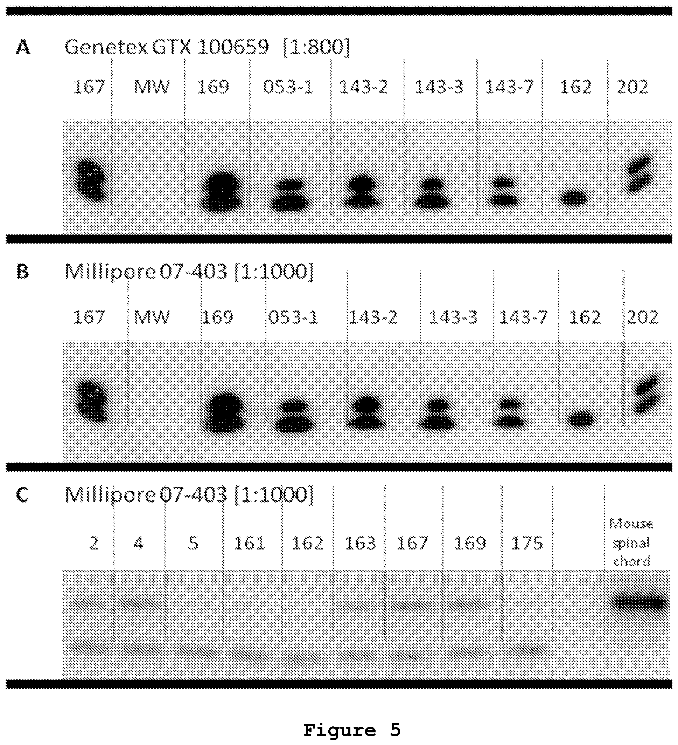

- **FIG. 5****: WB on stillborn piglets spinal cord.** Lane [A] displays results obtained with Genetex GTX 100659 at the 1:800 dilution, while lane [B] shows those obtained with Millipore 07-403 at the dilution 1:1000 on the same samples. Sample 162 corresponds to spinal cord homogenate from non-transgenic pig, used as negative control. In lane [C] in possible to appreciate a WB analysis conducted with Millipore 07-403 at the dilution 1:1000 on spinal cord from other piglets and on spinal cord homogenate from hSOD1-G93A mouse, used as positive control. In all hSOD1-G93A piglet samples it is possible to appreciate two lanes, corresponding to the two SOD1 isoforms: the endogenous swine protein displaying a lower molecular weight (16 KDa) and the human transgenic one, with a higher molecular weight (20 KDa).

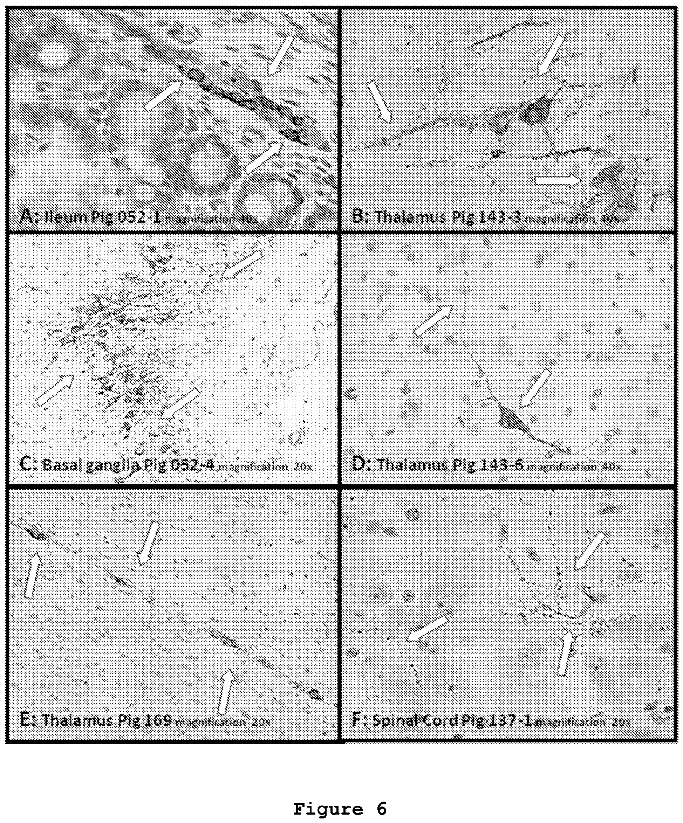

- **FIG. 6****: Genetex GTX 100659 IHC on FFPE piglet samples.** In figure A it is possible to appreciate hSOD1-G93A staining in the ileum with clusters of positive cells next to the *muscolaris mucosa.* Figures B and C show deposition pattern at the level of area *Hypothalamica lateralis.* In the same area (figure D), it is also possible to appreciate isolated cells displaying hSOD1-G93A staining. Figure E shows a bundle of positive cells lining the optic tract. Figure F displays deposition pattern in the spinal cord: it is possible to appreciate granular aggregates along fibres.

### DETAILED DESCRIPTION OF THE INVENTION

The present invention created an animal model, consisting in a transgenic swine genetic line carrying the human SOD1 gene with mutation G93A responsible for the onset of ALS that is useful in studying such a disease and to develop novel diagnostic markers and therapeutic approaches for the human species.

Transgenic swine have been produced by means of many techniques such as pronuclear microinjection, sperm-mediated gene transfer and lentiviral transgene delivery.

These techniques present, nevertheless, some disadvantageous aspects. As a matter of fact, pronuclear microinjection is very inefficient (Clark and Whitelaw, 2003), sperm-mediated gene transfer is attractive due to its simplicity, but suffers from high variability and generates transgenic animals with a non uniform expression of the transgene (Lavitrano et al., 2002; WO-A-2007/124751) and although lentiviral transgene delivery offers an efficient method for the generation of transgenic pigs (Whitelaw et al., 2004), the use of lentivirus (HIV-1; EIAV) pose many safety and ethical concerns, due to the proven ability to activate oncogenes as well as the possibility of re-acquiring their pathogenic characteristics.

Therefore, the present invention used a different technique with respect to the previous techniques used for generating ALS animal models: *in vitro* transfection of cultured somatic cells combined with Somatic Cell Nuclear Transfer (SCNT).

The inventors constructed a vector capable of promoting in the swine species, preferably pigs, more preferably mini-pigs, the expression of the mutated SOD1 protein, in a manner similar to what happens in the patient suffering from ALS.

To achieve this goal inventors were able to obtain a stable expression of exogenous gene (hSOD1-G93A) in a consistent, reproducible way and in the long term in living animals. In fact the inventors in a previous study (Chieppa et al. "Creation of a ubiquitous vector for expression of hSOD1G93A in pigs" Transgenic research 2010; 19:326) - using a different vector (carrying neomycin as selectable marker, to express the mutated gene) - found that only 22% of positive clones showed high and uniform transgene expression 25 days post-transfection, while the remaining positive clones showed variegate expression or transgene silencing.

In the present invention, using a puromycin as selectable marker, to express the mutated protein, inventors solved this problem. In particular thanks to the ability of the drug (puromycin) to select in a more consistent and effective way and shorter time the transgenic clones four days after transfection, inventors have been capable to select, during *in vitro* culture, suitable transgenic cell clones with the desired protein expression level.

Genetic transformation of animals generally results in a large and random variation in transgene expression between individual transformants. This variability has been attributed to the positioning effect that is the presence of different transgene integration sites and copy numbers.

Since integration of foreign DNA seems to occur randomly in the animal genome, it has been assumed that some transgenes would be integrated into transcriptionally inactive regions, while others would be integrated into relatively transcriptionally active regions (Park et al., 2002).

To solve such a random insertion of the transgene into the swine genome the inventors decided to introduce the chicken lysozyme an element (known as 5'MAR), which is located upstream of the chicken lysozyme gene and comprises a region of approximately 3 kb (Phi-Van and Stratling, 1996), in their pCAGGS constructs.

The combination of puromycin selection cassette, with accurate screening of the cell clones for the desired characteristics in combination with SCNT turn out to be successful and the inventors managed to produce a large number of piglets, five of which are alive and adult. All pigs generated in this way express hSOD1-G93A.

The vectors that were used to produce hSOD1-G93A transgenic pigs proved to be very effective to obtain an ubiquitous expression in somatic cells and in the tissues of the animals generated by SCNT.

The inventors decided to use in SCNT experiments a pool of donor cells showing different transgene expression level in order to minimize the risk of using cell clones unable to generate a viable animal. As a matter of fact, since this is the first hSOD1-G93A swine model produced so far, no data are available about the toxicity related to transgene expression level in the early stage of porcine embryonic development and after embryo transfer in sows.

Since donors cells with different expression levels have been employed in SCNT, the cloned piglets present variable transgene expression levels depending on the particular cell from which they have been created.

However, transgenic swine could be re-cloned from the cells cryopreserved after birth from best expressing animal, in a successive SCNT experiment in order to further standardize the hSOD1-G93A pig model or could be bred to sows to produce F1 generation, of which accordingly Mendelian law 50% will inherit the disease.

The SOD1 protein is an enzyme with antioxidant function by reducing the superoxide ion (O²⁻) level, a toxic free radical product during the oxidative cellular metabolism. The superoxide ion is capable of altering proteins, membranes and DNA. Precisely the involvement of a mutated protein in its pathogenesis leads to include ALS in the proteinophaty family. Studies on transgenic rodent models was aimed at understanding the mechanisms by which mutated SOD1 gene leads to the onset of ALS: they have ruled out that the motor neuron degeneration is the result of loss of dismutase activity and allowed to detect the formation of aggregates of ubiquitinated proteins in tissues affected, among other containing the mutant SOD1: it is assumed that these inclusions protein play a role in the interruption of cell functions damaging mitochondria, proteasomes, mechanisms of protein folding or other proteins.

Unlike rodent models that show an extremely high expression transgene level and a rapid disease course (Bendotti and Carri, 2004), the instant swine model presents an expression level comparable to that of human patients, were a single allele mutation results in aforementioned toxic gain of function.

Piglets expressing the hSOD1-G93A protein, already express the mutated protein at birth, are expected to show the full degeneration of upper and lower motor neurons, leading to muscle weakness, atrophy and evolving to complete paralysis, with times and modalities similar to those that occur in ALS patients. On one hand this could result in a longer pre-clinical phase and in an increase of animal maintaining costs, on the other hand the present hSOD1-G93A swine represents an invaluable opportunity to find early biomarkers and a closer and more faithful model to reproduce human pathology since ALS is typically an adult-onset disease.

Currently, an animal model recapitulating all the ALS crucial aspects has not yet been produced.

However, since increasing difficulties are emerging in translating information gleaned from rodent models into therapeutic options for ALS patients, there is an urgent need for an intermediate research system. The present inventors do believe that a swine model provides this essential bridge between insights gained from rodent models and the reality of treating a human disease.

### Materials and Methods

### Animal experiments

All procedures involving animals and their care are conducted in conformity with national and international regulations (EEC Council Directive 86/609, OJL358, 1, 12 December 1987; Italian Legislative Decree 116/92, Gazzetta Ufficiale della Repubblica Italiana 10, 18 February 1992; and Guide for the Care and Use of Laboratory Animals, U.S. National Research Council, 1996) and after the approval of LTR-Avantea Local Ethic Committee. Yucatan Black fibroblasts (PAF) were obtained from adult boar ear biopsy, coming from Italian pig farming. Subsequently PAF have been used in cloning procedures to obtain wild type pigs.

### Vector construction

The inventors have previously developed an ubiquitous EGFP expression vector, driven by the pCAGGS hybrid promoter (CMV-IE enhancer + chicken beta actin promoter) (Niwa et al., 1991) which is characterized by maintaining high expression level through the F1 generation of pigs (Brunetti et al., 2008).

A Destination Vector pMGOrfA5'3'MARpuro5171 was created inserting the Multisite Gateway system (Invitrogen) Conversion cassette (OrfA) into the ubiquitous expression vector. The resulting vector carried the pCAGGS promoter inserted between two insulators (5' MAR of chicken lysozyme gene) (McKnight et al., 1992) to prevent various silencing effects (positional or copy number effects). The structure was also provided with a floxed, then removable using the Cre recombinase, SV40-Puro cassette to select the transfected clones.

The *Bam*HI/*Xho*I fragment of hSOD1G93A cDNA obtained by restriction of pcDNA3.0hSOD1-G93A plasmid was inserted into the pENTRL1L2Oligo*Sac*I*Sal*I vector, obtaining pENTRL1L2-hSOD1-G93A.

The SalI-BamHI fragment was removed from the construct and the resulting pENTRL1L2-hSOD1-G93AdelSB was used, after sequencing, in a LR exchange reaction with the Destination Vector pMGOrfA5'3'MARpuro5171. This exchange reaction, mediated by the LR Clonase, was used to transform chemically competent *E.coli* cells (One Shot Mach1-Invitrogen). The resulting pMG5'3'MARPuro5171-hSOD1G93A vector (Figure 1 and 2 - SEQ ID No.:1) was purified with Plasmid Mini Kit (Qiagen, Hilden, Germany), analyzed by different restriction enzymes, confirmed by sequencing (Figure 2) and finally linearized by ApaLI (Fermentas). After phenol/chlorophorm purification, the vector was precipitated and re-suspended in TE buffer.

### Cell isolation and culture

Primary porcine fibroblasts cultures were recovered from adult male ear biopsy (pig adult fibroblasts, PAF). Biopsy specimens were cut in small pieces with a scalpel blade and the resulting tissue pieces were distributed on the surface of gelatin-coated dishes containing 1.5 ml of DMEM/TCM199 with 20% of fetal bovine serum (FBS). Culture medium was changed every 3 days.

Cells were allowed to grow until they reached 50% of confluence. Tissue pieces were then removed and the cells sub-cultured until they reached confluence in DMEM/TCM199 with 10% of FBS and growth factors (bFGF). Growth conditions consisted of a 38°C temperature and of an atmosphere composed by 90% N₂, 5% O₂ and 5% CO₂.

Exponentially growing cultures were cryopreserved in DMEM/TCM199 with 20% FBS and 10% DMSO and stored in liquid nitrogen. These batches of cells were used throughout the following experiments.

### Transfection of hSOD1-G93A vector into adult fibroblasts

The day before transfection, passage 3 PAF were trypsinized, counted, and plated into 60 mm dishes in order to obtain about 1x10⁶ cells at 80% confluency in 24 hours. On the transfection day, cells were trypsinized counted and resuspended in 100 µl of nucleofector solution (Basic Nucleofector Kit, Prim. Fibroblasts; Amaxa, Cologne, Germany), mixed with 5 µg of the linearized pMG5'3'MARPuro5171-hSOD1-G93A vector. PAF and linearized vector were then transferred into the nucleofection cuvettes and transfected with V-24 program (Nucleofector Amaxa).

After nucleofection cells were plated in 60-mm culture dishes containing fresh culture medium. After 24 hours, the drug (Puromycin: 1 µg/ml) employed in colonies selection was added. After 4 days, puromycin-resistant colonies were picked up using 5-mm cloning discs and transferred into 24 mm well dishes. Cells were then expanded in DMEM/TCM199 with 10% FBS and 5 ng/mL of bFGF at a temperature of 38.5°C and in a humified atmosphere containing 5% CO₂ and 5% O₂.

An aliquot was cryopreserved as described above, to be employed in nuclear transfer and the remaining cells were subcultured to perform expression analysis.

### Immunocytochemistry (ICC)

Cells (transgenic hSOD1-G93A PAF and primary culture cells from ear biopsy obtained from transgenic piglets) were fixed with 4% PFA and then stored in phosphate buffer (PB) until analysis.

In order to detect the expression of human SOD1 by immunocytochemistry (ICC) the inventors used a rabbit polyclonal antibody (07-403 Millipore, concentration 1:200), directed against the full-length wild-type hSOD1 plus an N-terminal methionine.

Unspecific binding blocking (10% goat serum) was followed by primary antibody incubation (RT, two hours). After 3 washes (PB with 0.2% BSA and 0.05% saponine, 3 minutes each) a secondary FITCH-conjugate antibody incubation (1 h at RT) was performed. Nuclei were counterstained with Hoechst (RT, 15'). After two washes slides were finally mounted in mounting medium (Citifluor). We used Human Umbilical Vein Endothelial Cells (Huvec) and wild type PAFs respectively as positive and negative controls. Fluorescence was detected by exposing fibroblasts to an epifluorescent light mounted on an inverted microscope (Nikon TE-DH100W) equipped with a FITC (for green) and DAPI (for nuclei) filter and with a digital imaging system (Nikon DIGITAL SIGHT DS-L1). Subjective assessment of transgene expression level was based on the comparison of the fluorescence intensity revealed in PAF clones with that of the controls. The negative control was assigned a Score=0 while the positive a Score=2, as showed in Figure 3. It should be noted that this is as subjective scores attribution and values presented in this study are not objectively measured, neither absolute.

### Preparation of nuclear donor cells and SCNT

Transgenic 1A1, 1A2, 1B1, 1C2, 1D1, 1D2, 1E2, 2A2, 2A6, 2B2, 2C1 and 2C3 hSOD1-G93A-fibroblasts clones were selected, as nuclei donors, according to uniformity and intensity of their expression level.

The day before nuclear transfer, donor cells were induced into quiescence by serum starvation (0.5% FBS). 30 minutes before nuclear transfer, cells were prepared by trypsinization, washed and resuspended in SOF (Tervit et al., 1972) supplemented with 25 mM HEPES (H-SOF). Ovaries with corpora lutea were collected at local slaughterhouse and carried to laboratory at 31-33°C. Oocytes were aspirated from follicles larger than 3 mm in diameter, washed, and transferred to maturation.

DMEM-F12 supplemented with 10% FBS, 110 µg/ml sodium pyruvate, 75 µg/ml ascorbic acid, 100 µg/ml glutamine, 5 µg/ml myoinositol, 0.4 mM cystine, 0.6 mM cysteamine, ITS liquid media supplement (insulin, transferrin, selenite, Sigma, 1 µl/ml), gonadotropins (0.05 IU/ml FSH, and 0.05 IU/ml LH; Pergovet 75, Serono), 100 ng/ml long IGF1 (recombinant insulin growth factor I analog), 50 ng/ml long EGF (recombinant epidermal growth factor analog), and 5 ng/ml bFGF (human recombinant) was used as maturation medium.

Oocytes were cultured at 38.5°C in 5% CO₂ in humidified atmosphere. After 42 hours maturation, oocytes were denuded of cumulus cells by vortexing in the presence of hyaluronidase in HSOF and returned to maturation medium. Only oocytes displaying extruded polar body were selected. NT-embryos were reconstructed following a zona-free method (Lagutina et al., 2005, 2006; Oback et al., 2003). The zona pellucida of oocytes with extruded polar body was digested with 0.5% pronase in PBS. The oocytes were washed in H-SOF with 10% FBS and returned to maturation medium. All the following manipulations were performed in H-SOF with 10% FCS. Zona-free oocytes were exposed to cytochalasin B (5 µg/ml) and Hoechst (5 µg/ml) for 5 min prior to enucleation. Metaphase chromosomes were removed under very short exposure to UV light with a blunt enucleation pipette.

After enucleation, zona-free cytoplasts were individually washed for few seconds in 300 µg/ml phytohemagglutinin P in PBS and then quickly dropped over a single donor cell (Vajta et al., 2003) settled at the bottom of a microdrop of the diluted donor cell suspension.

Forty-six to forty-eight hours after maturation onset, formed cell couples were washed in 0.3 M mannitol (Ca⁺⁺-free, 100 µM Mg⁺⁺) solution, fused by double DC-pulse of 1.2 kV/cm applied for 30 µsec and returned to maturation medium. 2 hours later (after 48-50 hours of maturation), NT embryos were activated by double DC-pulses of 1.2 kV/cm for 30 µsec applied in 0.3 M mannitol solution, containing 1 mM Ca⁺⁺ (Cheong et al., 2002) and 100 µM Mg. After activation, embryos were kept in culture maturation medium with 5 µg/ml cytochalasin B for 4 hours.

NT embryos were cultured in SOF supplemented with essential and nonessential amino acids and with 4 mg/ml BSA (SOFaa) in a Well-of-the-Well system (WOW) modification (Vajta et al., 2000). During embryo culture half of the medium was renewed on day 3 (D+3) and on day 5 (D+5) with fresh SOFaa.

### Synchronization of sows, surgical embryo transfer, pregnancy diagnosis, and parturition

Estrus was synchronized by feeding 12 mg of alternogest (Regumate, Intervet, Peschiera Borromeo, Italy) per sow for 15 days and injecting 0.15 mg of PgF2a (Dalmazin, Fatro, Ozzano Emilia, Italy) on the 15th day of regumate treatment and 1000 IU of hCG (Chorulon, Intervet) 96 hours after the last altrenogest administration.

The SCNT embryos were transplanted to the sows uterus on D+5 of development. Embryo transplantation was performed 4 days after animals ovulation by midventral laparatomy and pregnancy was examined at day 29, 36, 50, and 62 by ultrasonography. A cesarean delivery was performed at D+114 of gestation.

### Tissue banking

Ear biopsy was performed on all piglets, in order to obtain primary PAF cultures and subsequent cryopreservation to establish a cell bank from each individual piglet. Brain, spinal cord, peripheral nerves, muscles and organs were sampled from stillborn or euthanized animals. Tissue samples were either formalin-fixed and paraffin-embedded (FFPE) or paraformaldehyde-fixed.

After 24 hours, paraformaldehyde samples were rinsed 2 times in PBS and then exposed to growing sucrose concentrations in order to achieve cryoprotection. Finally, samples were kept for 30 minutes in a 1:1 30%sucrose: OCT solution and then included in OCT, frozen in isopentane and stored at - 80°C.

Finally, a part of every tissue was snap-shot freezed in isopentane and stored at -80°C.

### Western Blot (WB)

Cells (transgenic hSOD1-G93A PAF and primary culture cells from ear biopsy obtained from transgenic piglets) were lysed using Laemmli buffer 1X containing β-mercaptoethanol (5%) and boiled for 10 minutes. Total protein was quantified by Qubit fluorometer (Invitrogen) using the QuantIT Protein quantification kit (Invitrogen).

Tissues obtained from stillborn piglets were homogenized (1:5 mg/ml) in lysis buffer (50mM Tris HCl pH8, 150mM NaCl, 5mM EGTA pH8, 1.5mM MgCl₂, 10% anhydrous glycerol, 1% Triton, 100µg/ml (=0.57mM) PMSF). Protein quantification was performed by BCA Protein Assay kit (Pierce).

Thirteen µg of each sample were loaded onto Glicine-SDS-PAGE (4-12%) and electrophoretically separated for 45 minutes at 200V using the MiniproteanII chamber (Biorad). Blotting onto Immun-Blot PVDF membrane (Biorad) was obtained after 1h at 100V using MiniproteanII electroblotter (Biorad) according to manufacturer protocol. The resulting PVDF membranes were subsequently processed following the instructions of the chemiluminescent detection system Lumi-LightPLUS Western Blotting Kit Mouse/Rabbit (Roche). Detection of transgenic target was achieved using either a polyclonal Antibody 07-403 (1:1000 Millipore) or a polyclonal Antibody GTX 100659 (1:800 Genetex) that is specifically directed against hSOD1 region within amino acids 75 and 138, which is different between human and swine. Pig β-actin expression was detected using the mAb ab6276 (1:5000 Abcam). The treated membranes were finally exposed, developed and fixed.

### Immunohistochemistry (IHC)

All FFPE tissues were analysed by IHC. After antigen retrieval (95° bain-marie for 20 minutes), sections were treated with 3% hydrogen peroxide in methanol for 10 minutes to block endogenous peroxidase. Sections were incubated in Normal Goat Serum for 20 minutes and then overnight at 4°C in the primary antibody (GTX 100659; 1:250) dissolved in PBS.

Afterwards, sections were rinsed 3 times in PBS and incubated with relevant secondary antibody (1:200) for 1 hour. After 3 rinses in PBS, sections were incubated with Avidin Biotin Complex for 30 minutes. DAB was used as revealing agent. Sections were counterstained with haematoxylin.

Ubiquitine-immunoreactive misfolded protein was detected by the same IHC protocol, but the antigen retrieval step, with a rabbit polyclonal antibody (Dako Z0458, 1:100).

### Results

### Transfection of hSOD1-G93A vectors into adult fibroblasts and related analysis.

The transfection experiments conducted with the pMG5'3'MARPuro-hSOD1-G93A vector have led to the isolation of 26 PAF clones, which were analysed by WB and ICC. All clones, listed in Table 1, revealed different degrees of transgene expression, to which a score was assigned. Four clones showed a transgene expression level lower than the control one (score=2), and three clones achieved the same score as the control. The remaining 19 clones showed a higher expression (Score ranging from 3 to 6).

**Table 1**

| **Clone** | **Score** |
|---|---|
| 1A1 | 3 |
| 1A2 | 6 |
| 1A4 | 1 |
| 1A5 | 5 |
| 1B1 | 3 |
| 1B3 | 4 |
| 1B6 | 6 |
| 1C1 | 1 |
| 1C2 | 2 |
| 1C6 | 4 |
| 1D1 | 5 |
| 1D2 | 1 |
| 1D5 | 4 |
| 1D6 | 5 |
| 1E1 | 6 |
| 1E2 | 4 |
| 2A1 | 6 |
| 2A2 | 6 |
| 2A3 | 5 |
| 2A4 | 1 |
| 2A5 | 4 |
| 2A6 | 4 |
| 2B2 | 4 |
| 2C1 | 5 |
| 2C2 | 5 |
| 2C3 | 4 |

### Somatic Cell Nuclear Transfer (SCNT)

Six SCNT experiments were conducted. In the first and second experiment, a pool of clones (1A1, 1C2, 1D2 and 1E2) was used as a nucleus donor. In the third and fourth experiment a pool composed of 1B1, 1D1, 2C1 and 2B2 clones was employed, while a pool composed of 1A2, 2A2, 2A6 and 2C clones was employed in the fifth and sixth experiment. All the SCNT experiments conducted are listed in Table 2 where viable embryo percentages (ranging from 35.38% to 50.50%) were obtained. Table 2 contains the following information: The "ID clones" column shows the names of hSOD1-G93A PAF clones that composed the cells pool, used as nuclei donors. In "'N° SCNT" column the number of SCNT experiments is reported. The "average score" column indicates the transgene expression level owned by the pool of PAF clones. "N" is the number of pairs cytoplasts/PAF formed after the fusion. "Cl" is the number of segmented embryos and its value is compared to the total percentage of reconstructed embryos. "'Mc/Bl" indicates the number of compact morulae and blastocysts at the sixth day of in vitro culture. "Tot embryo" indicates the number of viable embryos, with the relative percentage value obtained by comparison with the initial number of manipulated oocytes. Piglets indicate the pregnancy outcome, with the number of vital and stillborn piglets obtained.

**Table 2**

| ***ID clones*** | **N° SCNT** | **Av. Sc.** | **N** | **Cl** | % | **Mc/Bl D6** | **TOT emb ryo** | % | **Piglets Vital/ Stillborn** |
|---|---|---|---|---|---|---|---|---|---|
| A1, E2, C2, D2 | 1 | 2 | 195 | 181 | 94,66 | 8 mc, | 69 | 35,38 | No pregnancy |
| A1, E2, C2, D2 | 2 | 2 | 204 | 185 | 92,82 | 11 mc, 63 bl | 74 | 36,27 | No pregnancy |
| B1, 2B2, 2C1, D1 | 3 | 3 | 200 | 172 | 86,00 | 21 mc, | 75 | 37,50 | 6/4 |
| B1, 2B2, | 4 | 3 | 209 | 151 | 72,25 | 31 mc, 47 bl | 78 | 37,32 | 5/2 |
| 1A2, 2A6, 2A2, 2C3 | 5 | 5 | 205 | 179 | 87,32 | 96 bl | 96 | 46,83 | 3/1 |
| 1A2, 2A6, 2A2, 2C3 | 6 | 5 | 202 | 178 | 88,12 | 102 bl | 102 | 50,50 | 2/7 |

### Development to term of SCNT embryos derived from adult hSOD1-G93A fibroblasts

The transfer of 494 embryos to six recipient sows resulted in four pregnancies. The 4 pregnant sows received 75, 78, 96, 109 embryos obtained from, the third, fourth, fifth and, sixth SCNT experiment respectively. Pregnancies were carried to term and resulted in the birth of 10, 7, 4 and 9 piglets with a mean efficiency of blastocyst development to term of 8,78%. Among produced piglets 16 were alive and 12 were stillborn. Five piglets survived artificial hand raising, weaning and developed normally. The remaining 11 piglets died within 48-96 h from birth due to events commonly reported in commercial herds (i.e. some piglets were not nursing, others developed diarrhoea, pneumonia etc).

### Detection of hSOD1-G93A expression in tissues from stillborn piglets and living animals

Fibroblasts obtained from ear biopsy of both living and stillborn piglets, were analyzed by ICC and revealed a transgene expression level comparable with that of PAF used as nuclei donors (Figure 4).

Furthermore, IHC and WB analysis were performed on dead and stillborn piglets tissues. Snap shot spinal cord samples were homogenised and analysed by WB. Transgenic protein was revealed using both antibodies (07-403 Millipore and GTX 100659 Genetex). In Figure 5 it is possible to appreciate two lanes, corresponding to the two SOD1 isoforms: the endogenous swine protein displaying a lower molecular weight (16 KDa) and the human transgenic one, with a higher molecular weight (20 KDa).

Immunohistochemistry (Figure 6) was performed on coronary sections of all FFPE samples. Analysis performed by GTX 100659 revealed granular mutant protein aggregates in neurites and perikarya in brain (from area *hypothalamica lateralis* to the third ventricle), spinal cord (especially at the thoracic level), peripheral nerves (brachial plexus and sciatic nerve) and in the Enteric Nervous System.

The same brain areas also revealed ubiquitine immunoreactivity characterized by intracytoplasmatic aggregates.

Naturally, while the principle of the invention remains the same, the details of construction and the embodiments may vary widely with respect to what has been described and illustrated by way of example, without departing from the scope of the present invention.

### REFERENCES

Benatar M. Neurobiol Dis. 2007 Apr;26(1):1-13.
Bendotti C, Carri MT. Trends Mol Med, 2004. 10(8):393-400.
Brunetti et al. Cloning Stem Cells. 2008 Dec;10(4):409-19.
Bugos et al. Cell Mol Neurobiol. 2009 Sep;29(6-7):859-69.
Cheong et al. Mol Reprod Dev. 2002 Apr;61(4):488-92.
Clark and Whitelaw. Nat Rev Genet. 2003 Oct;4(10):825-33.
Gordon et al. Lancet Neurol. 2007 Dec;6(12):1045-53.
Lagutina et al. Reproduction. 2005 Oct;130(4):559-67.
Lagutina et al. Cloning Stem Cells 2006 Winter;8(4):283-93.
Lavitrano et al. Proc Natl Acad Sci U S A. 2002 Oct 29;99(22):14230-5.
McKnight et al. Proc Natl Acad Sci U S A. 1992 Aug 1;89(15):6943-7.
Niwa et al. Gene 1991 Dec 15;108(2):193-199
Oback et al. Cloning Stem Cells 2003;5(1):3-12.
Park et al. Biol Reprod. 2002 Apr;66(4):1001-5. Phi-Van and Stratling. Biochemistry. 1996 Aug 20;35(33):10735-42.
Schnabel J. Nature. 2008 Aug 7;454(7205):682-5.
Scott et al. Amyotroph Lateral Scler. 2008;9(1):4-15
Tervit et al. J Reprod Fertil. 1972 Sep;30(3):493-7.
Turner and Talbot. Prog Neurobiol. 2008 May;85(1):94-134.
Vajta et al. Mol Reprod Dev. 2000 Mar;55(3):256-64.
Vajta et al. Biol Reprod. 2003 Feb;68(2):571-8.
Van de Bosch L. J Biomed Biotechnol. 2011:348765.
Whitelaw et al. FEBS Lett. 2004 Jul 30;571(1-3):233-6.

## Claims

1. Transgenic swine comprising a vector having the nucleotide sequence set forth in SEQ ID No.: 1 encoding a human Cu/Zn superoxide dismutase gene (SOD1) carrying a glycine to alanine mutation at codon 93, wherein the transgenic swine is suitable to develop amyotrophic lateral sclerosis.

2. Transgenic swine according to claim 1, wherein the transgenic swine carries the exogenous gene in a single allele.

3. Transgenic swine according to claims 1 or 2, wherein the transgenic swine expresses the exogenous gene in form of granular mutant SOD1 aggregates in brain, spinal cord, peripheral nerves and enteric nervous system.

4. Transgenic swine according to any one of claims 1 to 3, wherein the transgenic swine expresses ubiquitin intracytoplasmatic aggregates in brain.

5. Transgenic swine according to any one of claims 1 to 4, wherein the transgenic swine is a pig, preferably a mini-pig

6. A process for generating a transgenic swine comprising an exogenous gene encoding a human Cu/Zn superoxide dismutase gene (SOD1) carrying a glycine to alanine mutation at codon 93, the process comprising the steps of:
a. providing a vector encoding the exogenous gene;
b. providing adult swine fibroblasts;
c. nucleofecting the vector into the fibroblasts, obtaining transgenic fibroblasts;
d. providing at least one enucleated swine oocyte by means of metaphase chromosomes removal from an intact oocyte;
e. contacting the at least one enucleated oocyte with at least one transgenic fibroblast;
f. fusing the at least one enucleated oocyte with at least one transgenic fibroblast obtaining an embryo;
g. cultivating the embryo;
h. implanting at least one cultivated embryo, preferably 70, more preferably 120, to a sow uterus;
i. natural birth or cesarean section delivering of at least one newborn transgenic swine at the end of sow pregnancy,
wherein after step c. analysis of vector expression in transgenic fibroblasts is carried out for selecting transgenic fibroblasts having uniform and intensive expression of the vector, wherein step e. is carried out using the selected transgenic fibroblasts, wherein the vector encoding the exogenous gene is carrying the selectable marker Puromycin, and wherein the vector encoding the exogenous gene has the nucleotide sequence set forth in SEQ ID No.:1.

7. Process according to claim 6, wherein before step e. a pooling of more than two transgenic fibroblasts is carried out, and step e. is carried out by contacting the at least one enucleated oocyte with the pool of more than two transgenic fibroblasts.

8. Use of the transgenic swine according to any one of claims 1 to 5 for screening compounds pharmaceutically active in the treatment of amyotrophic lateral sclerosis.

9. Use of the transgenic swine according to any one of claims 1 to 5 as an animal model for the study of amyotrophic lateral sclerosis.

## Patentansprüche

1. Transgenes Schwein, umfassend einen Vektor mit der in SEQ ID No: 1 dargelegten Nucleotidsequenz, die ein humanes Cu/Zn-Superoxiddismutase-Gen (SOD1) kodiert, welches eine Glycin zu Alanin Mutation an Codon 93 trägt, wobei das transgene Schwein dazu geeignet ist, amyotrophe Lateralsklerose zu entwickeln.

2. Transgenes Schwein gemäß Anspruch 1, wobei das transgene Schwein das exogene Gen in einem einzigen Allel trägt.

3. Transgenes Schwein gemäß den Ansprüchen 1 oder 2, wobei das transgene Schwein das exogene Gen in Form granulärer Aggregate von mutantem SOD1 im Gehirn, im Rückenmark, in peripheren Nerven und im enterischen Nervensystem exprimiert.

4. Transgenes Schwein gemäß einem der Ansprüche 1 bis 3, wobei das transgene Schwein intrazytoplasmatische Ubiquitin-Aggregate im Gehirn exprimiert.

5. Transgenes Schwein gemäß einem der Ansprüche 1 bis 4, wobei es sich bei dem transgenen Schwein um ein kleineres Schwein, vorzugweise ein Minischwein handelt.

6. Verfahren zum Erzeugen eines transgenen Schweins, umfassend ein exogenes Gen, das ein humanes Cu/Zn-Superoxiddismutase-Gen (SOD1) kodiert, welches eine Glycin zu Alanin Mutation an Codon 93 trägt, wobei das Verfahren die Schritte umfasst:
a. Bereitstellen eines Vektors, der das exogene Gen kodiert;
b. Bereitstellen adulter Schweine-Fibroblasten;
c. Nucleofizieren des Vektors in die Fibroblasten, wobei transgene Fibroblasten erhalten werden;
d. Bereitstellen mindestens einer entkernten Schweine-Oozyte mittels Metaphasechromosomen-Entfernung aus einer intakten Oozyte;
e. In-Kontakt-Bringen der mindestens einen entkernten Oozyte mit mindestens einem transgenen Fibroblasten;
f. Fusionieren der mindestens einen entkernten Oozyte mit mindestens einem transgenen Fibroblasten, wobei ein Embryo erhalten wird;
g. Züchten des Embryos;
h. Implantieren des mindestens einen gezüchteten Embryos, vorzugsweise 70, stärker bevorzugt 120, in den Uterus einer Sau;
i. natürliche Geburt oder Kaiserschnitt, wobei am Ende der Trächtigkeit der Sau mindestens ein neugeborenes transgenes Schwein auf die Welt gebracht wird;
wobei nach Schritt c. zum Selektieren transgener Fibroblasten mit gleichförmiger und intensiver Expression des Vektors eine Analyse der Vektorexpression in transgenen Fibroblasten ausgeführt wird, wobei Schritt e. unter Verwendung der selektierten transgenen Fibroblasten ausgeführt wird, wobei der Vektor, der das exogene Gen kodiert, den selektierbaren Marker Puromycin trägt und wobei der Vektor, der das exogene Gen kodiert, die in SEQ ID No: 1 dargelegte Nucleotidsequenz aufweist.

7. Verfahren gemäß Anspruch 6, wobei vor Schritt e. ein Pool von mehr als zwei transgenen Fibroblasten erstellt wird und Schritt e. durch In-Kontakt-Bringen der mindestens einen entkernten Oozyte mit dem Pool von mehr als zwei transgenen Fibroblasten ausgeführt wird.

8. Verwendung des transgenen Schweins gemäß einem der Ansprüche 1 bis 5 zum Screenen von Verbindungen, die bei der Behandlung von amyotropher Lateralsklerose pharmazeutisch wirksam sind.

9. Verwendung des transgenen Schweins gemäß einem der Ansprüche 1 bis 5 als Tiermodell zur Untersuchung von amyotropher Lateralsklerose.

## Revendications

1. Suidé transgénique comprenant un vecteur ayant la séquence nucléotidique représentée dans la séquence SEQ ID No : 1 codant pour un gène de Cu/Zn superoxyde dismutase humain (SOD1) portant une mutation de glycine en alanine au niveau du codon 93, où le suidé transgénique est approprié au développement de la sclérose latérale amyotrophique.

2. Suidé transgénique selon la revendication 1, dans lequel le suidé transgénique porte le gène exogène dans un seul allèle.

3. Suidé transgénique selon la revendication 1 ou 2, dans lequel le suidé transgénique exprime le gène exogène sous forme d'agrégats granulaires de SOD1 mutant dans le cerveau, la moelle épinière, les nerfs périphériques et le système nerveux entérique.

4. Suidé transgénique selon l'une quelconque des revendications 1 à 3, dans lequel le suidé transgénique exprime des agrégats intracytoplasmiques d'ubiquitine dans le cerveau.

5. Suidé transgénique selon l'une quelconque des revendications 1 à 4, dans lequel le suidé transgénique est un porc, de préférence un mini-porc.

6. Procédé de génération d'un suidé transgénique comprenant un gène exogène codant pour un gène de Cu/Zn superoxyde dismutase humain (SOD1) portant une mutation de glycine en alanine au niveau du codon 93, le procédé comprenant les étapes qui consistent :
a. à fournir un vecteur codant pour le gène exogène ;
b. à fournir des fibroblastes porcins adultes ;
c. à nucléofecter le vecteur dans les fibroblastes, en obtenant des fibroblastes transgéniques ;
d. à fournir au moins un ovocyte porcin énucléé par l'élimination de chromosomes en métaphase d'un ovocyte intact ;
e. à mettre en contact l'au moins un ovocyte énucléé avec au moins un fibroblaste transgénique ;
f. à fusionner l'au moins un ovocyte énucléé avec au moins un fibroblaste transgénique en obtenant un embryon ;
g. à cultiver l'embryon ;
h. à implanter au moins un embryon cultivé, de préférence 70, plus préférablement 120, dans l'utérus de truie ;
i. à aider à la mise bas naturelle ou par césarienne d'au moins un suidé transgénique nouveau-né à la fin de la gestation de la truie,
où après l'étape c. l'analyse de l'expression de vecteur dans des fibroblastes transgéniques est réalisée pour sélectionner des fibroblastes transgéniques ayant une expression uniforme et intensive du vecteur, où l'étape e. est réalisée en utilisant les fibroblastes transgéniques sélectionnés, où le vecteur codant pour le gène exogène porte le marqueur sélectionnable Puromycine, et où le vecteur codant pour le gène exogène a la séquence nucléotidique présentée dans la séquence SEQ ID NO : 1.

7. Procédé selon la revendication 6, dans lequel avant l'étape e., un regroupement de plus de deux fibroblastes transgéniques est réalisé, et l'étape e. est réalisée en mettant en contact l'au moins un ovocyte énucléé avec le groupe de plus de deux fibroblastes transgéniques.

8. Utilisation du suidé transgénique selon l'une quelconque des revendications 1 à 5 pour cribler des composés pharmaceutiquement actifs dans le traitement de la sclérose latérale amyotrophique.

9. Utilisation du suidé transgénique selon l'une quelconque des revendications 1 à 5 comme modèle animal pour l'étude de la sclérose latérale amyotrophique.
